# DEMANDE DE BREVET EUROPEEN

(11) **EP 0 685 556 A1**
(43) Date de publication de la demande: **06.12.1995**
(21) Numéro de dépôt: 95401170.6
(22) Date de dépôt: 19.05.1995
(51) Int. Cl.: C12N 5/06

(54) **Véhicule aqueux pour micro-organismes non autonomes du règne animal à maintenir vivants hors de leur milieu naturel, notamment spermatozoides de bovin**

(30) Priorité: 31.05.1994 FR 9406586
(71) Demandeur: INSTRUMENTS DE MEDECINE VETERINAIRE, F-61300 l'Aigle (FR)
(72) Inventeur: Ghazarian, Chaqué, F-69210 Saint-Bel (FR); Cassou, Bertrand, F-61300 L'Aigle (FR); Bousseau, Serge, F-61300 L'Aigle (FR); Brillard, Jean-Pierre, F-37300 Joue Les Tours (FR)
(74) Mandataire: CABINET BONNET-THIRION

(57) **Abrégé**

Le véhicule de l'invention est conçu pour des micro-organismes non autonomes du règne animal, tels que gamètes et embryons qu'il faut maintenir vivants hors de leur milieu naturel en vue d'interventions de l'homme, et est susceptible d'être conservé prêt à l'emploi sur des périodes prolongées. Il s'agit d'un milieu aqueux comprenant, outre notamment des agents de nutrition (sucres et acides aminés), des tampons et des sels minéraux utilisés classiquement, un produit protecteur formé comme support de croissance embryonnaire par un organisme vivant, qui, en l'état de la technique, est d'origine animale et ajouté extemporanément à l'emploi du véhicule et qui, selon l'invention, est une lécithine extraite de graines de soja et introduite dans le milieu aqueux à la formulation du véhicule. Lorsque la conservation des micro-organismes est cryogénique, on ajoute au véhicule du glycérol. Dans une application particulière, les micro-organismes sont des spermatozoïdes de bovins.

## Description

L'invention se rapporte à un véhicule pour micro-organismes non autonomes du règne animal, tels que gamètes et embryons, à maintenir vivants en dehors de leur milieu naturel en vue d'interventions de l'homme, susceptible d'être conservé prêt à l'emploi sur des périodes prolongées, ce véhicule étant un milieu aqueux comprenant, outre notamment des agents de nutrition, des tampons et des sels minéraux, un produit protecteur formé comme support de croissance embryonnaire par un organisme vivant et qui, en l'état de la technique est d'origine animale et ajouté extemporanément à l'emploi du véhicule.

Au sens de la présente description, par micro-organismes non autonomes, on entendra des organismes unicellulaires ou paucicellulaires, eucaryotes ou procaryotes, vivants mais non doués de capacité de reproduction immédiate, c'est-à-dire de reproduction d'organismes descendants au même stade d'évolution que leurs ascendants directs. Ainsi des gamètes ou des embryons ne sont pas autonomes car ils ne peuvent engendrer directement des gamètes ou des embryons. Par contraste, les amibes constituent des micro-organismes autonomes.

La conservation de gamètes, embryons et cellules du règne animal vivants hors de leur milieu naturel, à l'état frais et surtout congelé pour des interventions vétérinaires ou connexes, notamment pour la fécondation artificielle, nécessite des véhicules liquides, qui, en termes de métier, sont dits "dilueurs" ou "conservateurs" pour les spermatozoïdes, et milieux pour les ovocytes, embryons et cellules. Ces liquides comportent, en milieu aqueux, des agents de nutrition tels que sucres et amino-acides, des tampons, éventuellement des anticoagulants, ainsi que, pour des conservations cryogéniques des micro-organismes, un inhibiteur de formation de cristaux de glace, tel qu'un polyol, notamment glycérol. On y ajoute, au moment de l'emploi, un produit protecteur de membranes cellulaires d'origine animale tel que sérums et albumines ; très souvent ce produit protecteur est pour les dilueurs du jaune d'oeuf dont le pouvoir protecteur est particulièrement accentué du fait, semble-t-il, que ce jaune d'oeuf constitue le support essentiel de croissance de l'embryon de poulet, donc d'un organisme animal fragile à un stade où la division cellulaire est particulièrement intense, et les réactions de défense aux agressions extérieures encore peu développées. Dans la pratique on utilise, soit du jaune d'oeuf frais, soit des préparations industrielles liquides destinées notamment à l'industrie alimentaire.

Ces produits protecteurs sont, en contrepartie de l'efficacité de la protection membranaire, favorables à la multiplication de micro-organismes, pathogènes ou non leur conservation à l'état efficace est aléatoire, de sorte que systématiquement, ils ne sont ajoutés au véhicule qu'au moment de l'emploi.

Mais cet ajout au moment de l'emploi est une opération qu'il est très difficile d'exécuter dans des conditions d'asepsie rigoureuse, d'autant que la stérilisation des produits protecteurs est très délicate, car elle ne doit, ni détériorer le produit protecteur lui-même (les produits contenant des albuminoïdes coagulent à la chaleur, par exemple), ni laisser subsister dans le produit protecteur stérilisé des composés toxiques pour les micro-organismes à conserver.

L'objectif de l'invention est donc de créer un véhicule pour micro-organismes non autonomes du règne animal conservés en vie, susceptible d'être conservé sur des périodes prolongées prêt à l'emploi, c'est-à-dire contenant dès la formulation le produit protecteur, et stérilisé.

Cela est obtenu avec un véhicule pour micro-organismes non autonomes du règne animal, tels que gamètes et embryons, à maintenir vivants en dehors de leur milieu naturel en vue d'interventions de l'homme, susceptible d'être conservé prêt à l'emploi sur des périodes prolongées, ce véhicule étant un milieu aqueux comprenant, outre notamment des agents de nutrition, des tampons et des sels minéraux, un produit protecteur formé comme support de croissance embryonnaire par un organisme vivant, et qui, en l'état de la technique, est d'origine animale et ajouté extemporanément à l'emploi du véhicule, caractérisé en ce que ledit produit protecteur est une lécithine extraite de graines de soja, et introduite dans le milieu aqueux à la formulation du véhicule.

Il a été constaté que, de façon imprévisible, la lécithine extraite de graines de soja, bien qu'issue du règne végétal et formant partie du support de développement du germe d'une graine, présentait un pouvoir protecteur comparable à celui des protecteurs usuels à l'égard des membranes de cellules appartenant au règne animal, sans en partager la fragilité devant les processus de stérilisation, et la mauvaise conservation dans le temps, ce qui permettait de l'incorporer au véhicule lors de la formulation de celui-ci, et de stériliser le véhicule avec tous ses composants. Dans ces conditions, le véhicule pouvait être conservé à la température de réfrigérateur (typiquement 4°C) pendant des périodes prolongées, atteignant au moins six mois, sans détérioration décelable.

De préférence, pour la conservation cryogénique des micro-organismes non autonomes, le véhicule contiendra, à dose efficace, un polyol apte à inhiber la formation de cristaux de glace, typiquement glycérol. L'utilisation d'un tel polyol, classique en conservation cryogénique, s'est avérée compatible avec le véhicule de l'invention.

De préférence également, le véhicule est formulé avec une quantité d'eau réduite, et on le dilue pour l'emploi avec de l'eau stérile. On réduit ainsi le volume du véhicule pendant sa conservation entre sa formulation et son emploi ; la disposition d'eau stérile, et la dilution du véhicule concentré sans affecter la stérilité du véhicule final ne présentent aucune difficulté particulière comparable avec la pratique antérieure d'ajout extemporané du produit protecteur.

En disposition préférée, un véhicule conçu spécialement pour du sperme de bovin comprend, à l'état concentré, pour 200 ml d'eau :
- Triméthylol-méthylamine: 3,4 g à 4,2 g
- Citrate trisodique dihydraté: 13,7 g à 16,75 g
- Chlorure de potassium: 0,55 g à 0,67 g
- Fructose: 1,65 g à 2,0 g
- Glucose: 0,68 g à 0,84 g
- Lactose: 0,41 g à 0,50 g
- Lactate de calcium: 0,09 g à 0,11 g
- Glycine: 5,15 g à 6,25 g
- Glycérol: 64 ml à 78 ml
- Lécithine de soja: 6,75 g à 8,25 g

ce véhicule étant à diluer, pour l'emploi, avec 750 ml à 900 ml d'eau.

Des caractéristiques secondaires, et les avantages de l'invention ressortiront d'ailleurs de la description qui va suivre à titre d'exemple.

### Exemple 1 - Véhicule pour sperme de bovin

On a formulé un véhicule en dissolvant ou dispersant dans 200 ml d'eau stérile 3,809 g d'un tampon Triméthylolméthylamine (Tampon Tris), 15,238 g de citrate trisodique dihydraté anticoagulant, 0,609 g de chlorure de potassium, des sucres, soit 1,828 g de fructose, 0,761 g de glucose et 0,457 g de lactose, 0,100 g de lactate de sodium, 5,714 g d'un amino-acide, ici glycine, 71 ml de glycérol et 7,5 g de lécithine de soja. Pour l'emploi, on portera la quantité d'eau à 1 025 ml par ajout de 825 ml d'eau stérile.

Après formulation, le véhicule concentré est stérilisé de façon classique, mis en flacon étanche et conservé dans un réfrigérateur à + 4°C.

### Exemple 2 - Emploi du véhicule

En laboratoire, on a réalisé des congélations de spermatozoïdes de taureaux, chaque éjaculat étant divisé en deux parts ; une part a été introduite dans un véhicule conforme à l'exemple 1 après dilution, et l'autre part a été introduite dans un dilueur de formulation propriété de la Demanderesse incluant du lait et du jaune d'oeuf. De façon classique, on a ajouté à chaque part, dans des proportions équivalentes, des antibiotiques usuels. Après quoi les deux parts ont été mises séparément dans des tubes dits paillettes qui ont été scellés et immergés dans l'azote liquide, suivant un processus classique.

### Exemple 3 - Contrôle in vitro de la qualité de conservation des spermatozoïdes

Un nombre égal de paillettes appartenant à l'une et l'autre part d'éjaculats de plusieurs taureaux (pour réduire l'influence de l'individualité), et choisies au hasard ont été sorties de l'azote liquide et décongelées pour un examen microscopique, en déterminant les pourcentages de spermatozoïdes motiles et progressifs. Ces pourcentages se sont révélés du même ordre pour les dilueurs classique et selon l'invention, avec un avantage pour le dilueur selon l'invention; mais cet avantage n'était pas significatif en termes de probabilité.

### Exemple 4 - Contrôle par fécondation in vitro

On a choisi comme à l'exemple 3 deux lots de paillettes appartenant respectivement à l'une et l'autre part, pour féconder in vitro des ovocytes prélevés sur des vaches, et l'on a décompté le nombre d'ovocytes fécondés ainsi que le nombre d'embryons parvenus au stade blastocyste. Là encore les pourcentages de réussite se sont avérés sensiblement égaux pour les deux lots, avec un avantage pour le lot comprenant le dilueur de l'invention. Toutefois, compte tenu de la dispersion résultant de la diversité des ovocytes, l'avantage n'était pas significatif en termes de probabilité.

On observera que les exemples précédents étaient destinés à vérifier que le dilueur de l'invention était au moins aussi efficace que les dilueurs classiques, et ne pouvaient faire ressortir un avantage important de l'invention, qui est d'opérer en routine en conditions stériles. En effet, d'une part, les essais de laboratoire peuvent être exécutés avec plus de soins et dans une ambiance mieux définie qu'un travail de caractère industriel, et d'autre part, les contaminations qui risquent d'être engendrées par les processus classiques n'apparaissent pratiquement pas aux stades précoces entourant la fécondation.

Sous un autre aspect, on a vérifié que les concentrations en constituants du véhicule dans une plage de ± 10 % autour des valeurs indiquées à l'exemple 1, ne dégradent pas sensiblement l'efficacité des véhicules vis-à-vis des spermatozoïdes. On observera que le glycérol et la lécithine contribuent dans une moindre part que les autres composants à la compatibilité biologique du véhicule pour les micro-organismes non autonomes, le glycérol étant de ce point de vue sensiblement neutre et n'agissant que pour éviter que la congélation ne provoque un éclatement des membranes, tandis que la lécithine apparaît comme un protecteur extérieur aux micro-organismes.

Par ailleurs, on a fait des essais de composition de milieux pour la conservation d'ovocytes en vue de fécondation "in vitro", et de culture d'embryons avant implantation dans l'utérus de femelles, en partant de compositions classiques de milieux et en y ajoutant, à la formulation, de la lécithine de soja en substitution aux produits protecteurs usuels ajoutés extemporanément à l'emploi, avec des résultats, du point de vue maintien de la vie des micro-organismes non autonomes, au moins comparables aux résultats obtenus classiquement, tandis que les conditions d'asepsie des processus étaient nettement améliorées.

Bien entendu, l'invention n'est pas limitée aux exemples décrits, mais en embrasse toutes les variantes d'exécution dans le cadre des revendications.

## Revendications

1. Véhicule pour micro-organismes non autonomes du règne animal, tels que gamètes et embryons, à maintenir vivants en dehors de leur milieu naturel en vue d'interventions de l'homme, susceptible d'être conservé prêt à l'emploi sur des périodes prolongées, ce véhicule étant un milieu aqueux comprenant, outre notamment des agents de nutrition, des tampons et des sels minéraux, un produit protecteur formé comme support de croissance embryonnaire par un organisme vivant, et qui, en l'état de la technique, est d'origine animale et ajouté extemporanément à l'emploi du véhicule, caractérisé en ce que ledit produit protecteur est une lécithine extraite de graines de soja, et introduite dans le milieu aqueux à la formulation du véhicule.

2. Véhicule selon la revendication 1, prévu pour une conservation cryogénique des micro-organismes non autonomes, caractérisé en ce qu'il contient, à dose efficace, un polyol apte à inhiber la formation de cristaux de glace.

3. Véhicule selon l'une des revendications 1 et 2, caractérisé en ce que, pour la conservation entre formulation et emploi, le véhicule, concentré, comporte une quantité partielle d'eau, un complément d'eau étant à ajouter pour l'emploi.

4. Véhicule selon la revendication 3, conçu spécialement pour du sperme de bovin, caractérisé en ce qu'il comprend, à l'état concentré, pour 200 ml d'eau :
Triméthylol-méthylamine 3,4 g à 4,2 g
Citrate trisodique dihydraté 13,7 g à 16,75 g
Chlorure de potassium 0,55 g à 0,67 g
Fructose 1,65 g à 2,0 g
Glucose 0,68 g à 0,84 g
Lactose 0,41 g à 0,50 g
Lactate de calcium 0,09 g à 0,11 g
Glycine 5,15 g à 6,25 g
Glycérol 64 ml à 78 ml
Lécithine de soja 6,75 g à 8,25 g
ce véhicule étant à diluer, pour l'emploi, avec 750 ml à 900 ml d'eau.

5. Véhicule selon la revendication 4, caractérisé en ce qu'il contient sensiblement, pour 200 ml d'eau :
Triméthylol-méthylamine 3,809 g
Citrate trisodique dihydraté 15,238 g
Chlorure de potassium 0,609 g
Fructose 1,828 g
Glucose 0,761 g
Lactose 0,457 g
Lactate de calcium 0,100 g
Glycine 5,714 g
Glycérol 71 ml
Lécithine de soja 7,5 g
ce véhicule étant à diluer, pour l'emploi, avec sensiblement 825 ml d'eau.
